# FASCICULE DE BREVET EUROPEEN

(11) **EP 0 365 421 B1**
(45) Date de publication et mention de la délivrance du brevet: **23.03.1994**
(21) Numéro de dépôt: 89402862.0
(22) Date de dépôt: 17.10.1989
(51) Int. Cl.: C07J 1/00, C07B 59/00, A61K 43/00, A61K 31/565

(54) **Ligands spécifiques de récepteurs d'hormones stéroides estrogènes et progestagènes, application et produits intermediaires de synthèse**
Spezifische Liganden für Östrogen- und Progestagen-Steroidhomonrezeptoren, deren Verwendung und Synthesezwischenprodukte
Specific ligands for estrogenic and progestagenic steroidal hormone receptors, their use and synthesis intermediates

(30) Priorité: 19.10.1988 FR 8813737
(43) Date de publication de la demande: 25.04.1990
(73) Titulaire: LA REGION WALLONNE, B-1050 Bruxelles (BE)
(72) Inventeur: Zeicher, Marc, B-1180 Bruxelles (BE); Quivy, Jacques, B-1348 Louvain La Neuve (BE)
(74) Mandataire: Warcoin, Jacques

(56) Documents cités:
- EP-A- 0 137 434
- EP-A- 0 169 515
- FR-A- 2 268 528
- FR-A- 2 613 937
- STEROIDS, vol. 38, no. 5, novembre 1981, pages 537-555, San Francisco, US; T. RATAJCZAK et al.: "The synthesis and study of some potential affinity labeling reagents for estrogen receptors"

## Description

La présente invention concerne de nouveaux ligands stéroïdes spécifiques de récepteurs hormonaux estrogènes ou progestagènes.

Une application des ligands selon l'invention est la thérapie ciblée et/ou l'imagerie médicale, notamment du cancer. Ces ligands peuvent toutefois également être utilisés dans le dosage desdits récepteurs hormonaux.

La demande de brevet EP 310 645, comprise dans l'état de la technique au sens de l'article 54(3), décrit un composé 11 bêtachlorométhyl, 17alpha(E)-iodovinyl-oestradiol.

L'article de stéroïdes, vol. 38 n^{o} 5, 1981, pages 537-555 décrit un composé 11-bêta-chlorométhyl-oestradiol.

La demande de brevet EP 169 515 et 137 434 décrivant des composés 17alpha(Z)-iodovinyl-oestradiol."

Un but de la présente invention est de proposer des analogues d'hormones stéroïdes présentant une haute affinité pour le récepteur hormonal et qui se lie de manière quasi irréversible au récepteur en vue d'améliorer la proportion d'analogues fixés.

Un autre but de la présente invention est que les dérivés halogènés de ces analogues soient stables dans le milieu extracellulaire, notamment dans le plasma, comme dans le cytoplasme, tout en gardant une haute affinité pour le récepteur hormonal.

Soumises à ces dérivés, toutes les cellules contenant des récepteurs hormonaux, qu'elles soient malignes ou saines, peuvent être l'objet d'un accroissement de cytoxicité.

Toutefois, on observe quand même une sélectivité améliorée, puisque dans les traitements de chimiothérapie de cancer conventionnel, les tissus cibles estrogènes sains ont soit un taux de prolifération de cellules faibles, soit ne sont pas essentiels à la vie. Il en est de même pour les récepteurs progestagènes.

La présente invention a donc pour objet des ligands spécifiques de récepteurs d'hormones stéroïdes estrogènes ou progestagènes ayant pour formule 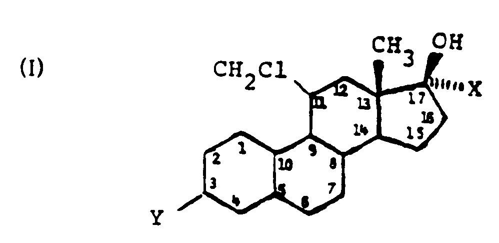
dans laquelle
- X représente un groupement vinyl substitué sur la double liaison selon une isomérie Z par un halogène radioactif ou non radioactif, et
- Y représente soit un groupe hydroxyle auquel cas le site sur lequel il est rattaché est un cycle aromatique, soit une fonction cétone auquel cas elle est conjuguée à une double liaison en C₄ - C₅.

Ces produits de formule I sont donc caractérisés en ce qu'ils comportent notamment :
- a: une fonction hydroxyle ou cétone en position C₃,
- b: une fonction chlorométhyle en beta sur la position C₁₁,
- c: une fonction vinyle en alpha sur la position C₁₇ et, soit
- d¹: un halogène quelconque non radioactif, à savoir l'iode, le fluore, le chlore ou le brome, lequel halogène est substitué sur la double liaison du groupement vinyl, selon une isomérie Z, soit
- d²: un isotope radioactif d'un halogène choisi parmi le fluore, l'iode, le brome et l'astatine substitué sur la double liaison du groupement vinyl selon une isomérie Z, notamment les isotopes ¹⁸F, ^{80m}Br, ²¹¹At, ¹²⁵I et ¹²³I.

Les fonctions en positions C₃ et C₁₇ confèrent à ces ligands une affinité élevée pour les récepteurs hormonaux auxquels ils sont destinés, à savoir des récepteurs, estrogènes ou progestagènes notamment.

La fonction 11 beta-chlorométhyle assure une liaison quasi "irréversible" entre le stéroïde et le récepteur, il s'agit en fait plutôt d'une très forte affinité pour le récepteur.

La formation du lien quasi "irréversible" entre le stéroïde et le récepteur améliore la stabilité du complexe et augmente sa concentration dans le noyau.

Les sites de liaison des hormones estrogènes et progestagènes présentent des homologies de séquences d'acide aminé, ce qui explique leur commun comportement s'agissant de la liaison irréversible entre le récepteur et le stéroïde en présence d'une fonction 11 betachlorométhyle.

Ces dérivés possédent une fonction vinyle en C₁₇ en alpha de la position C₁₇, ce qui leur confère une résistance au métabolisme et une liaison réduite aux protéines de liaison plasmatique.

Selon l'invention, l'halogène est situé sur la double liaison du vinyl en alpha sur la position C₁₇ selon une isomèrie Z. Ces ligands sont spécifiques des récepteurs estrogènes et progestagènes. Cette position a l'avantage de présenter une grande stabilité.

On peut citer en particulier comme ligands selon une première variante, ceux dont la fonction en C₃ est un groupe hydroxyl, le cycle sur lequel il est attaché étant un cycle aromatique. Ces ligands sont plus spécifiques des récepteurs estrogènes.

On peut également citer comme ligands selon une seconde variante de l'invention des ligands dont la fonction en position C₃ est une fonction cétone conjuguée à une double liaison en C₄-C₅. Ces ligands sont plus particulièrement spécifiques des récepteurs progestagènes.

Parmi les ligands, plus spécifiques des récepteurs estrogènes cités précédemment, on peut citer les 11 beta-chlorométhyl 17 alpha-halogénovinyl estradiol.

Parmi les ligands selon l'invention les plus spécifiques des récepteurs progestagènes, on peut citer particulièrement les 11 beta-chlorométhyl 17 alpha-halogéno vinyl 17 beta-hydroxy 1 9-nor 4-androstène 3-one.

Dans les ligands selon l'invention, l'halogène peut être non radioactif, les ligands seront alors utiles dans des dosages de récepteurs d'hormones stéroïdes, et pour tout usage thérapeutique où un effet oestrogénique ou progestatif puissant est requis.

Les dérivés oestrogéniques selon l'invention peuvent également être utilisés dans l'induction de récepteurs de progestatifs en vue de la thérapie ciblée du cancer à l'aide de progestatifs radioactifs.

Lorsque l'halogène est radioactif, les ligands peuvent être utiles aussi bien en imagerie médicale qu'en radiothérapie ciblée notamment du cancer mais également utiles comme réactifs dans le cas de dosage de récepteurs d'hormones stéroïdes.

Toutefois, les ligands marqués selon l'invention présentent un intérêt tout particulier dans le cas de la radiothérapie ciblée du cancer.

En effet, les hormones stéroïdes se lient avec une haute affinité aux récepteurs protéiques cytoplasmiques des cellules cibles et, après liaison, subissent une translocation dans le noyau cellulaire et activent la transcription des portions du génome relatives à l'effet physiologique propre à l'hormone. Etant donné le passage du complexe stéroïde-récepteur à proximité du DNA, un halogène radioactif porté par le stéroïde pourra endommager gravement le DNA et avoir un effet léthal sur la cellule cible.

Or, un certain nombre de cancers présentent une concentration élevée en récepteurs spécifiques soit aux estrogènes, soit aux progestagènes. Il s'agit notamment des cancers du sein, de l'utérus, de l'ovaire et de la Protaste. Par exemple, 65 % des cancers du sein présentent des niveaux détectables de récepteurs d'estrogènes (de 5000 à 50 000 molécules de récepteurs par cellule).

Un halogène radioactif attaché au stéroïde permettra la destruction spécifique des cellules cancéreuses. En outre, certaines permettront de visualiser la tumeur par radioimagerie. En fait, ces ligands constituent alors des agents de pilotage d'un élément actif supplémentaire qui est leur radionucléide.

Pour des sites de fixation intracellulaire tels que les récepteurs hormonaux, des analogues d'hormones présentant une haute affinité pour le récepteur et une faible affinité pour les protéines de liaison plasmitiques seront les agents de pilotage les plus appropriés.

L'utilisation de radioisotopes se désintégrant en émettant des électrons Auger est très propice en radiothérapie ciblée. Due au court parcours des électrons AUGER, l'efficacité de tels radioisotopes, en ce qui concerne l'inactivité cellulaire, est totalement perdue lorsqu'ils ne sont pas liés ou tout au plus à une distance de quelques atomes de l'ADN.

On a découvert selon l'invention que l'iode 123 qui a une demi-vie de 13, 21 heures et produit environ une vingtaine d'électrons AUGER par désintégration, a une demi-vie suffisamment courte pour ne pas exposer inutilement le patient à des doses de radioactivité pendant une période prolongée mais aussi suffisamment longue pour permettre la synthèse des produits radiopharmaceutiques et leur envoi par les centres de traitement situés loin du cylcotron de production, compte tenu du fait qu'il faut prévoir jusqu'à 48 heures de trajet et 24 heures de traitement.

Des ligands, plus particulièrement utiles pour la radiothérapie ciblée, comporteront donc l'isotope ¹²³I comme iode radioactif. Toutefois, le brome ^{80m}Br ou encore l'iode ¹²⁵I seront également acceptables en radiothérapie en tant qu'émetteurs d'électrons Auger.

Ces halogènes radioactifs ainsi pilotés présentent une forte activité cytotoxique, dans la mesure où ils sont amenés à proximité de l'ADN dont ils induisent des coupures dans la double hélice.

L'astate ²¹¹At, un émetteur de rayons alpha dont l'énergie est absorbée sur une distance d'environ 50 microns, ce qui correspond à quelques diamètres cellulaires, pourra être utilisé pour une radiothérapie permettant de tuer aussi les cellules avoisinantes des cellules ayant des récepteurs de stéroïdes.

Des ligands, plus particulièrement utiles pour l'imagerte médicale, notamment du cancer, porteront l'iode ¹²³I ou le fluore ¹⁸F.

Des ligands plus particulièrement utiles pour le dosage de réceptuers hormonaux porteront une iode ¹²⁵I.

Selon la présente invention, on a découvert que l'isomèrie Z de ladite fonction halogénovinyl notamment l'isomérie Z de la fonction 17 alpha-halogénovinyl et en particulier l'isomérie Z de la fonction 17 alpha-iodovinyl est préférable car elle confère aux ligands selon l'invention à activité estrogène ou progestagène une meilleure accumulation que l'isomérie E correspondante dans les tumeurs riches en récepteurs.

Les isomères Z sont moins hydrophobes que les isomères E et plus affines pour les récepteurs estrogènes ou progestagènes. Une hydrophobicité moins importante diminue en outre la fixation non spécifique dans le tissu graisseux qui est un facteur limitant pour l'emploi des hormones stéroïdes en imagerie.

La présente invention a enfin pour objet à titre de produit nouveau utile notamment comme produit intermédiaire pour la synthèse des analogues estrogènes et progestagènes selon l'invention, des dérivés de formule (I) dans laquelle un groupe tributylstannyl est substitué sur la double liaison du groupe vinyl rattaché en C₁₇ selon l'isomérie Z, à la place de l'halogène.

Un procédé objet de l'invention consiste à obtenir le dérivé halogéné à partir de ce dérivé tributylstannyl intermédiaire.

D'autres caractéristiques et avantages de la présente invention apparaîtront à la lumière de la description qui va suivre.

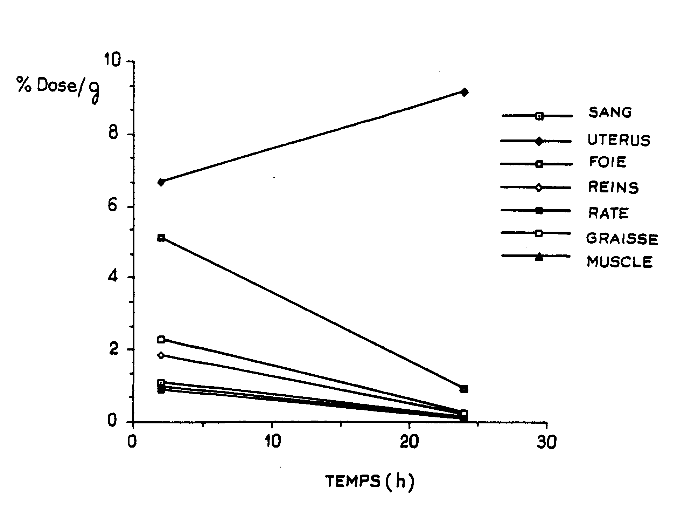

La description qui va suivre est fait en référence à la figure 1 qui représente l'accumulation de l'isomère Z du dérivé 16' (exemple 1) dans différents tissus de souris ovarectomisés BDF1 en fonction du temps.

### Exemple 1 : LIGANDS SPECIFIQUES DU RECEPTEUR ESTROGENE

Le schéma I ci-après reproduit une synthèse du ligand 11 beta-chlorométhyl-17 alpha-iodovinyl-estradiol.

L'étape 1 consiste en une préparation du Δ¹ adrénostérone 17 éthylène kétal à partir du Δ¹ adrénostérone (1').

25 g Δ¹ adrénostérone (84 mM) sont ajoutés sous forte agitation à un mélange de 500 ml de benzène, 25 ml d'éthylène glycol (environ 420 mM) et 1 g d'acide para-toluènesulfonique.

Le mélange est porté au reflux pendant 4 heures dans un appareil de Dean-Stark.

Le mélange réactionnel est extrait par 200 ml d'eau contenant 1 g de bicarbonate, puis par de l'eau saturée en sel, séché, puis évaporé.

On obtient une masse jaunâtre qui est lavée à chaud par 200 ml d'éther isopropylique.

Les cristaux blancs sont filtrés, puis séchés à 60°C, on obtient une masse de ± 26 g.

L'étape 2 consiste en la préparation du 11 β -hydroxy 17-éthylène kétal androsta 1,4-diène 3,17-dione (2').

340 g de (1') (88 mM) sont ajoutés sous azote à une suspension de 50 g (± 200 mM) dans 400 ml de tétrahydrofuranne.

Après 24 heures de réaction à température ordinaire, on ajoute 100 ml d'éther, puis 40 ml de NaOH 1N, puis 25 g de sulfate de sodium anhydre. On maintient l'agitation pendant la nuit.

Le mélange est filtré, puis le filtrat est évaporé sous vide.

La masse solide obtenue est lavée à chaud par 200 ml d'éther diisopropylique.

On obtient, après filtration et séchage, 24 g d'une poudre blanche (70,6 mM).

L'étape 3 consiste en la préparation du 3,11β-dihydroxy estra 1,3,5(10)-triène 17-one 17-éthylène kétal (3').

Sous atmosphère d'azote, un mélange de 3 g de lithium enrobé dans de l'huile (environ 0,4 M), 25 g de biphényl (0,16 M) et de diphénylméthane (0,08 M) dans 360 ml de tétrahydrofuranne est porté à reflux pendant 1 heure.

Le mélange réactionnel bleu foncé, auquel on ajoute 20,7 g de (2'), est à nouveau porté à reflux pendant 30 minutes.

Après refroidissement, on ajoute 8 ml de méthanol, puis 100 ml d'eau et on évapore le solvant sous vide.

Après redissolution dans la masse restante, dans l'éther, on extrait le produit formé par 300 ml de KOH 5 % qui est réacidifié avec l'acide acétique (12 ml).

Le précipité jaune est réextrait par l'acétate d'éthyle. Après évaporation du solvant, on redissout le produit à chaud dans 15 ml d'un mélange acétone et éther diisopropylique.

Après deux cristallisations, on obtient 10,5 g de produit.

Le point de fusion corrigé est : 191,1°C.

L'étape 4 consiste en la préparation du 3-benzyloxy, 11β-hydroxy estra 1,3,5(10)-triène 17-one 17-éthylène kétal (4').

Le mélange de 10,3 g de (3') (30 mM), 5,2 g de K₂CO₃ anhydre broyés et 100 ml de méthyléthyl cétone est porté à reflux sous forte agitation pendant 1 heure.

On ajoute alors 5,4 ml (45 mM) de bromure de benzyle et le reflux est maintenu pendant 48 heures.

Après extraction, on obtient 15 g d'une hui le jaunâtre.

Cette huile est engagée telle quelle dans la synthèse de (5').

L'étape 5 consiste en la préparation du 3-benzyloxy estra 1,3,5(10)-triène 11, 17-one 17-éthylène kétal (5').

30 mM de (4') brut (13 g) dissous dans 50 ml de chlorure de méthylène sec sont ajoutés en une fois au mélange de 13 g de pyridinium chlorochromate en suspension dans 200 ml de chlorure de méthylène sec.

Après 3 heures d'agitation à température ambiante, on ajoute 250 ml d'éther, ce qui entraîne la précipitation d'une masse noire.

Les solvants sont décantés, on lave l'insoluble avec 4 fois 50 ml du mélange v/v CH₂Cl₂/éther.

La phase organique est percolée sur une colonne de florisil, puis évaporée.

On obtient après purification sur silice 9,6 g d'une huile jaunâtre.

L'étape 6 est la préparation du 3-benzyloxy 11-hydroxy, 11-méthyltriméthylsilane estra 1,3,5(10)-triène 17-one 17-kétal (6').

8,6 g de (5') dissous dans 100 ml d'éther (20 mM) sont ajoutés rapidement à 165 ml d'une solution 1 M de chlorure de méthyltriméthylsilane de magnésium.

Le mélange est porté à reflux pendant 5 heures, puis décomposé et extrait.

Le produit brut est purifié sur silice, on obtient 5,7 g d'une huile visqueuse qui cristallise.

L'étape 7 est la préparation du 3-benzyloxy 11-méthylène 1,3,5(10)-estratriène 17-one (7').

Après addition de 0,5 ml d'HCL concentré, une solution de 5,2 g de (6') (10 mM) dans 50 ml d'acétone est agitée pendant 2 heures à température ambiante. Le mélange réactionnel est neutralisé par HCO₃⁻ concentré sous vide, extrait par CH₂Cl₂.

Le produit brut est recristallisé dans l'acétone.

On obtient 3,4 g de cristaux jaunâtres.

Le point de fusion corrigé est : 168,1°C.

L'étape 8 consiste en la préparation du 3-benzyloxy 11-méthylène 1,3,5(10)-estratriène 17-one, 17-kétal (8').

Une solution de 3 g de (7'), 7 mM, 100 ml de benzène, 3 ml de diéthylène glycol, 150 mg d'acide paratoluènesulfonique est refluée avec un appareil de "Dean Stark" pendant 4 heures.

L'extraction puis l'évaporation des solvants donnent 3,3 g d'une huile blanche qui est employée telle quelle.

L'étape 9 consiste en la préparation du 3-benzyloxy 11-hydroxyméthyl 1,3,5(10)-estratriène 17-one 17-kétal (9').

Une solution de 2,84 g de (8') (± 6 mM) dans 20 ml de THF sec est additionnée de 0,5 ml du complexe borane oxathiane (± 6 mM). Après 1 heure de réaction, on ajoute 6 ml d'éthanol, puis 4 ml de NaOH 3M (± 12 mM), enfin 10 ml de H₂O₂ 30 % (± 12 mM). Après une nuit à température ambiante, la réaction est extraite par CH₂Cl₂. On obtient une huile blanche qui cristallise rapidement (poids : 2,4 g).

L'étape 10 consiste en la préparation du 3-benzyloxy 11-chlorométhyl 1,3,5(10)-estratriène 17-one (10').

A une solution de 2 g de (9') d'environ 4 mM dans 20 ml de tétrahydrofuranne est ajoutée la suspension formée dans 30 ml de THF par la réaction de 2,1 g de triphénylphosphine (8 mM) et de 1,07 g (± 8 mM) de N-chlorosuccinimide.

Le mélange devenu limpide après à peu près 1 heure est laissé à température ambiante pendant la nuit. Le THF est évaporé sous vide, le résidu brut est redissout dans 50 ml d'acétone, auquel on ajoute 0,5 ml d'HCl concentré. On laisse sous agitation pendant 2 heures. L'extraction puis la purification sur silice donnent des cristaux blancs pour 1,02 g.

S.M. (FAB) 435 (M+1).

L'étape 11 consiste en la préparation du 3-benzyloxy 11-chlorométhyl 1,3,5(10)-estratriène 17α-éthynyl 17β-hydroxy (11').

A une solution de 0,93 g de (10') (à peu près 2 mM) dans 10 ml de tétrahydrofuranne, on rajoute 400 mg (± 4 mM) du complexe éthylènediamine - Acétylure de lithium. Après 4 heures de réaction, on réajoute 400 mg de réactif. On laisse réagir la nuit à température ambiante.

L'extraction par CH₂Cl₂ puis la purification sur silice nous donnent un solide légèrement jaunâtre d'environ 500 mg (1,02 mM).

L'étape 12 consiste en la préparation du 11 -chlorométhyl 3,17β-dihydroxy 17α-éthynyl 1,3,5(10)-estratriène (12').

440 mg de (11') 0,9 mM sont dissous à froid dans 20 ml de chlorure de méthylène.

On ajoute alors 4,5 ml (4,5 mM) d'une solution 1 M du complexe BF3/(CH₃)₂S.

Le mélange réactionnel est agité à froid pendant 45 minutes.

L'extraction puis la purification sur silice nous donnent une huile qui cristallise avec une masse d'environ 150 mg (0,375 mM).

L'étape 13 consiste en la préparation du dérivé (E)-11-chlorométhyl-3,17 β-dihydroxy-17 α -(2-tributylstannylvinyl)-1,3,5(10)-estratriène (13').

A une solution de 50 mg de (12') : 0,15 mM dans 1 ml de THF, on ajoute, sous N₂, 150 µl de tributylétainhydrure (à peu près 0,55 mM) et 5 mg de AIBN (à peu près 0,03 mM).

Le tube est hermétiquement fermé puis agité au bain d'huile à 70°C pendant 1 heure.

La réaction est extraite par un mélange acétate d'éthyle/eau.

L'huile brute est purifiée par silice ; on obtient 55 mg d'une huile blanche.

L'étape 14 consiste en la préparation du dérivé (E)-11-chlorométhyl-3,17β-dihydroxy-17α-(2-iodovinyl)-1,3,5(10)-estratriène (14').

A une solution de 32 mg de (13') (0,05 mM) dans 3 ml de CH₂Cl₂, on ajoute goutte à goutte une solution d'iode 0,1 mM dans CH₂Cl₂ jusqu'à persistance de la coloration rosée.

Après 30 minutes, on ajoute à peu près 10 ml d'H₂O avec un peu de NaHSO₃, on extrait par Et₂O.

Le produit brut est alors purifié sur silice ; on obtient 20 g de cristaux blancs pour une masse de 20 mg.

Données spectroscopiques :
RMN (CDcl₃, (CD₃)₂SO) S 1.1 (S,13CH₃), 3.45 (m, CHHcl), 3.62 (dd, CHHcl), 6.27 (d, CH=CHI, J=14 Hz), 6.53 (d, H(4)), 6.66 (dd, H(2)), 6.81 (d, CH=CHI, J=14 Hz), 7.03 (d, H(1)).

### Marquage à partir du dérivé tributyletain

50 µl d'une solution du dérivé (E)-17 alpha-tributylétainvinyl-11 beta-chlorométhylestradiol (13' du schéma 1 bis) dans l'éthanol absolu à 1 mg/ml sont ajoutés à 1 mCi de NaI¹²³ (10 µl dans H₂O pH 7-11) contenu dans un tube d'environ 500 µl avec bouchon à visser. On ajoute 10 µl d'une solution de chloramine-T à 1 mg/ml et on agite vigoureusement pendant 15 secondes. Ensuite, on ajoute 10 µl d'une solution de Na₂S₂O₅ à 2 mg/ml et 200 µl de tampon phosphate pH 7,4. Le mélange est passe sur cartouche SPE C18 (1 ML, BAKER) et le stéroïde est élué avec 1 ml d'éthanol. Après évaporation du solvant, le stéroïde marqué est purifié sur colonne HPCL µ BONDAPAK C18 avec phase mobile 50 % H₂O et 50 % d'acétonitrile. Le temps de rétention est de l'ordre de 17 minutes.

### C - SYNTHESE DE L'ISOMERE Z (SCHEMA 1 BIS)

L'étape 15 consiste en la préparation du dérivé (Z)-11 -chlorométhyl-3, 17 beta-dihydroxy-17 alpha-(2-tributylstannylvinyl)-1, 3, 5 (10)-estratriène (15').

105 mg (0,3 mmole) de dérivé (12'), 0,6 ml de HMPTA (hexamethyl phosphoretriamide, 0,3 ml de tributylétain hydrure (0,6 mmole) sont placés dans un tube à visser, sous argon.

Le mélange est mis en réaction pendant 60 heures sous forte agitation dans un bain d'huile à 70°C. Ensuite, on verse 5 ml d'eau et on extrait avec 3 x 5 ml d'acétate d'éthyle. Après évaporation du solvant, on obtient une huile blanchâtre qui est purifiée sur silice à l'aide d'un mélange CH₂Cl₂ et CH₃OH. On obtient 40 mg d'une huile blanche (23 %) ; 75 % du produit est récupéré.
TLC : 2 % CH₃OH dans CH₂Cl₂
Rf : 0,58.

L'étape 16 consiste en la préparation du dérivé (Z)-11 beta-chlorométhyl-3,17 beta-dihydroxy-17 alpha-(2-iodovinyl)-1, 3, 5 (10)-estratriène (16').

30 mg du dérivé (15') sont dissous dans 10 ml de CH₂Cl₂ ; on y ajoute une solution 1m de I₂ dans le CH₂Cl₂ jusqu'à persistance de coloration rose. On neutralise l'I₂ en excès par une solution d'H₂O bisulfite. Après extraction, on receuille le produit brut qui est purifié par chromatographie sur silice (CH₂Cl₂ 98 % éther 2 %). On obtient environ 15 mg de cristaux blancs.
TLC : 2 % CH₃OH dans CH₂Cl₂
Rf : 0,4
RMN : (CDCl₃° / 1,1 (s, 13CH₃), 3,45 (m, CHHCl), 3,58 (dd, CHHCl), 6,38 (d, CH=CHI, J=8Hz), 6,55 (d, H(4)), 6,65 (dd, H(2)), 6,85 (d, CH=CHI, J=8Hz), 7,05 (d, H(1)).

### Marquage du dérivé (16') à ¹²⁵I ou ¹²³I et stabilité de la solution radioactive

### Exemple avec Na¹²⁵I

La méthode est la même que pour le marquage de (14').

Le produit de départ est le dérivé (15').

Les conditions de séparation HPLC sont différentes : la phase mobile est : 60 % éthanol dans l'eau ; le débit est de 1 ml/min ; le produit final est collecté à 12 minutes. Le produit marqué est conservé tel quel dans l'éluat de la colonne (60 % éthanol). Dans ces conditions, il est stable au moins 12 jours à -20°C (déodination inférieure à 2 %).

### Hydrophobicité

L'isomère Z (16') est moins hydrophobe que l'isomère E (14') si l'on se réfère à un index d'hydrophobicité basé sur le temps de rétention de ces 2 produits en HPLC (phase inverse). Ainsi, avec un débit de 1 ml/min de 60 M éthanol dans H₂O sur une colonne microBondapack C18, l'isomère Z (16') a un temps de rétention de 12,1 minutes et l'isomère E (14') de 14,3 minutes.

Une hydrophobicité moins importante diminue la fixation non spécifique dans le tissu graisseux, fixation qui est un facteur limitant pour l'emploi des hormones stéroïdes en imagerie.

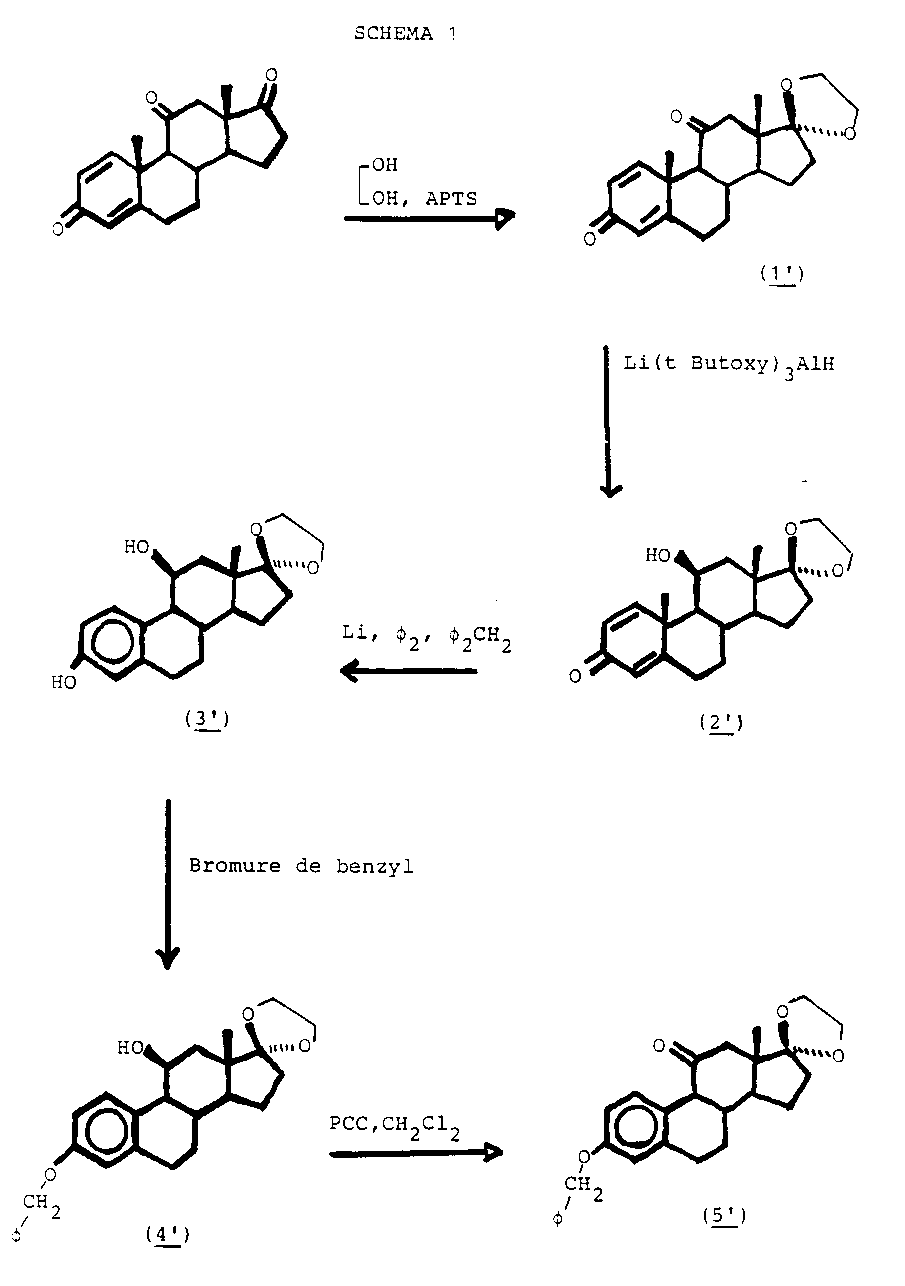

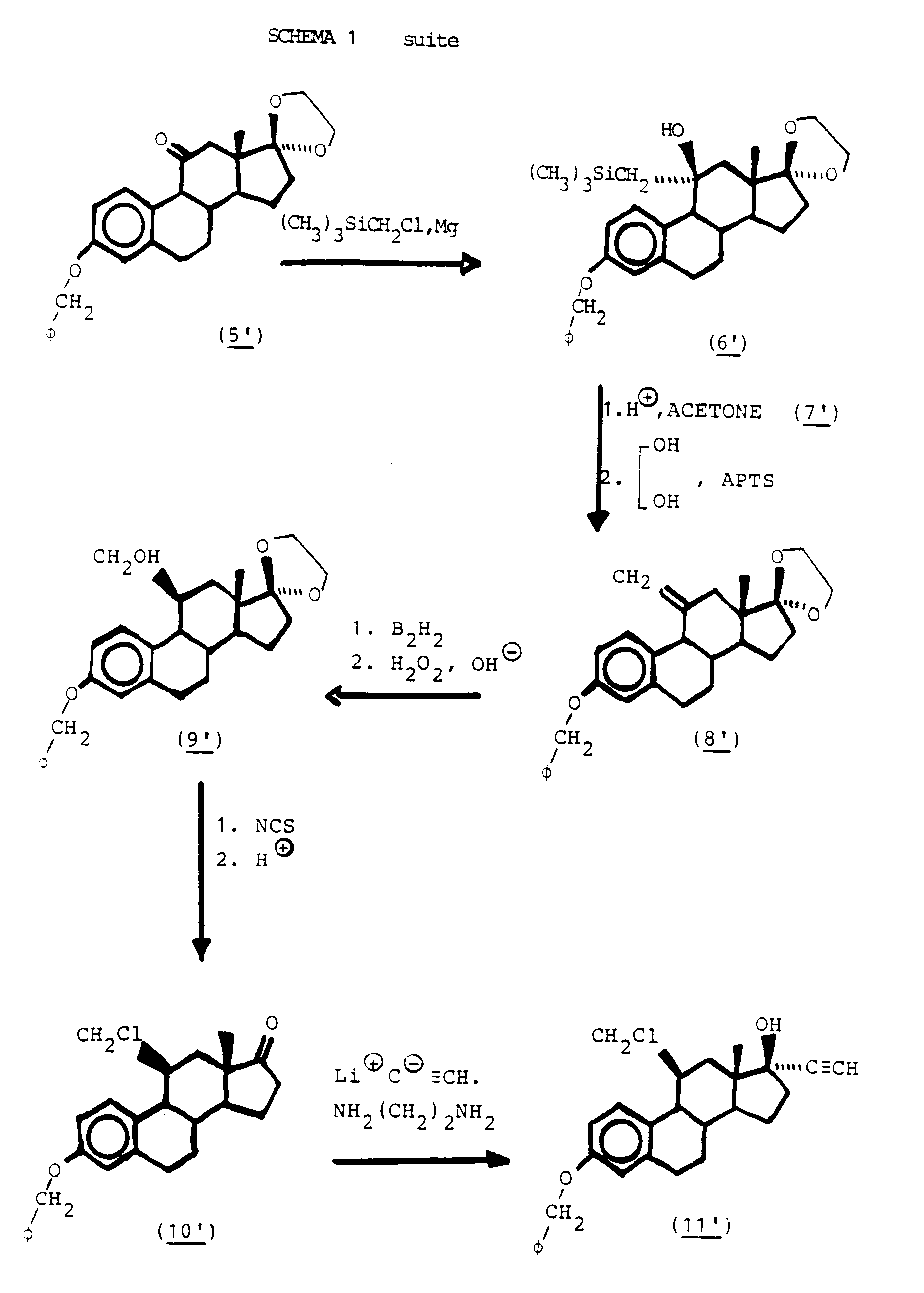

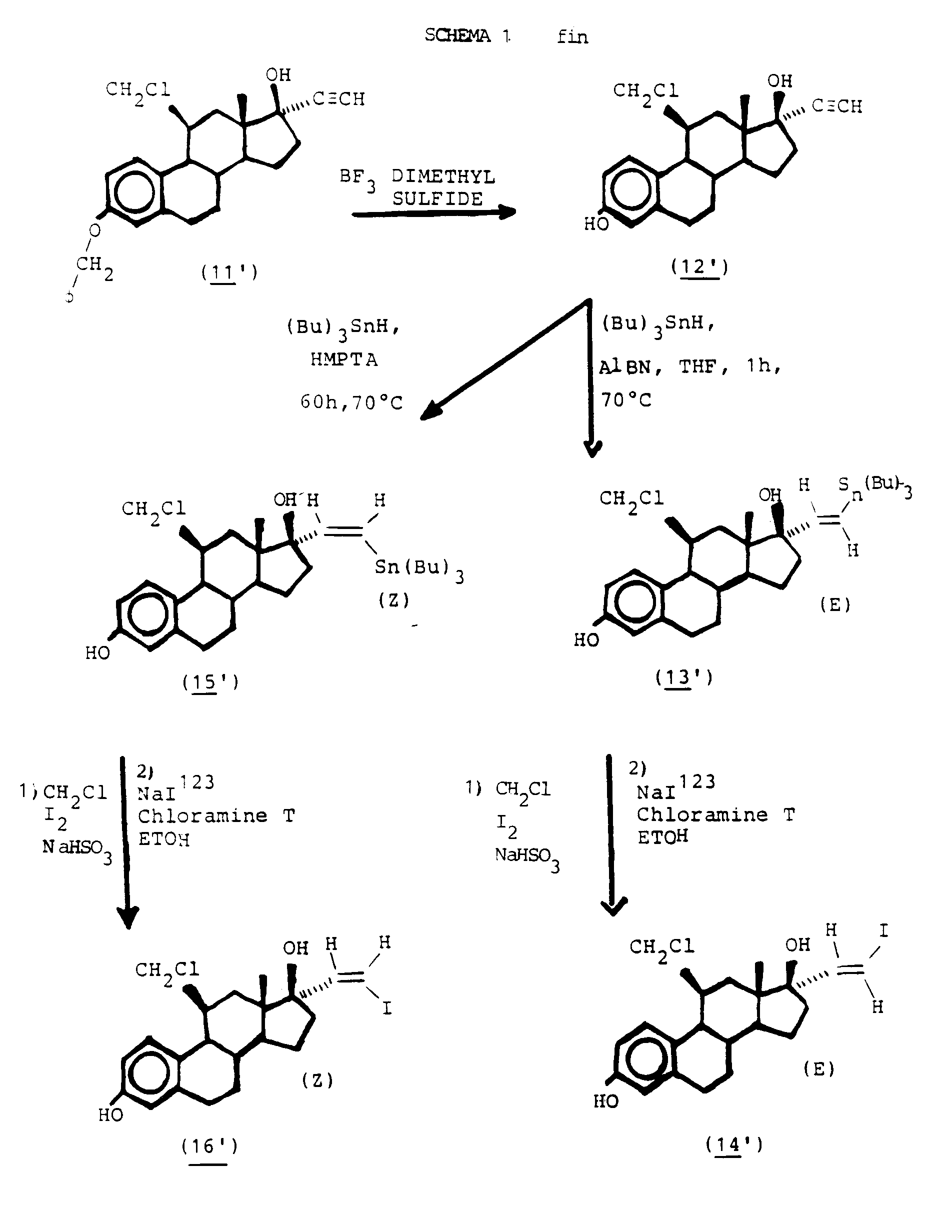

### Exemple 2 : BIODISTRIBUTION DE L'ISOMERE Z (16')

La biodistibution de l'isomère Z (16') dans des souris ovarectomisées BDF est très favorable.

8 souris BDF1 ovarectomisés ont été injectées avec 1 µci de 11 beta-chlorométhyl 17 alpha (2-iodovinyl) estradiol (¹²⁵I). 4 souris ont été sacrifiées 2 heures après l'injection et 4 à 24 heures après l'injection. Les pourcentages de doses injectées par gramme de tissu sont donnés au tableau 1 et représentés à la figure 1.

Il est remarquable que les rapports de concentrations utérus/sang, utérus/graisse et utérus/foie à 24 heures (reporté au tableau 2 ci-après et à la figure 1) soient de loin supérieurs à ceux décrits dans la littérature pour d'autres stéroïdes (par exemple, dans Hanson et al., Synthetis and Biodistribution of I-125 17 iodovinyl II ethylestradiol, in 7th International Symposium on Radiopharmaceutical Chemistry, Groningen, Netherlands, July 4-8, 1988 : dans des rats femelles immatures normales à 24 heures, les rapports donnés sont :
- utérus/foie = 3,89 ;
- utérus/poumon = 52,8 ;
- utérus/sang = 40,4.

Outre son intérêt pour l'hormonoradiothérapie, ce stéroïde 16' est très intéressant en tant qu'agent imagerie et comme réactif des dosages de récepteurs estrogènes.

**TABLEAU 1**

| TISSU | % dose injectée/g tissu (+S.D.) | |
|---|---|---|
| | temps : 2 heures | temps : 24 heures |
| Sang | 1.08 ± 0.24 | 0.14 ± 0.02 |
| Utérus | 6.66 ± 0.91 | 9.21 ± 1.44 |
| Foie | 5.10 ± 0.72 | 0.91 ± 0.05 |
| Reins | 1.84 ± 0.27 | 0.20 ± 0.02 |
| Rate | 0.97 ± 0.18 | 0.12 ± 0.01 |
| Graisse | 2.29 ± 0.25 | 0.26 ± 0.09 |
| Muscle | 0.89 ± 0.07 | 0.10 ± 0.02 |

**TABLEAU 2**

| RAPPORT | TEMPS : 2 h | TEMPS : 24 h |
|---|---|---|
| Utérus/sang | 6,16 | 65,80 |
| Utérus/muscle | 7,48 | 92,10 |
| Utérus/foie | 1,30 | 10,12 |
| Utérus/graisse | 2,90 | 35,42 |

### Exemple 3 : EMPLOI DU 11 BETA CHLOROMETHYL 17 IODOVINYL OESTRADIOL ¹²³I EN RADIOTHERAPIE CIBLEE

L'inhibition de la fixation d'oestradiol tritié sur des cytosols de tumeur humaine mammaire MCF7 riche en récepteurs oestrogéniques montre qu'à 25 °C, le 11 beta chlorométhyl 17 iodovinyl oestradiol a une affinité comparable au 11 beta chlorométhyl oestradiol et nettement supérieure à celle de l'oestradiol ("relative binding affinity" supérieure à 1000).

Vingt neuf souris BDF1 femelles ont été greffées à l'aide de tumeurs mammaires syngénéïques MXT implantées en sous-cutané. Quatorze souris ont reçu trois injections intraveineuses de 100 µCi de 11 beta chlorométhyl 17 iodovinyl ¹²³I d'isomérie E à deux semaines d'intervallé.

Les 100 µCi d'hormone marquée étaient injectés dilués dans 200 µl de solution contenant 9 gr/l NaCl, 5 % éthanol et 1 % de Tween 80. Les quinze souris contrôlées ont reçu aux mêmes dates 200 µl de la solution NaCl, éthanol Tween. Aucune toxicité aigüe n'a été décelée dans ces deux groupes sur une période de 60 jours.

Le groupe de souris traitées à l'hormone radioactive a montré un ralentissement significatif de la croissance tumorale par rapport au groupe témoin (inférieur à 45 % de la croissance tumorale moyenne au jour 18). Le groupe traité a aussi montré une meilleure survie.

Ces résultats démontrent un effet in vivo d'une hormonoradiothérapie ciblée à l'aide d'isotopes émetteurs d'électrons AUGER.

### Exemple 4 : INTERET DES PRODUITS FROIDS (16') ET (14') ET MARQUES A L'IODE 125 (DOSAGE DE RECEPTEUR DANS LES TUMEURS

Des essais de liaison au récepteur estrogène de cytosol d'utérus de veau (25°C, 1 h) à une dose saturante d'hormone marquée (16') à l'iode 125 (5mM final) montrent une liaison du même ordre que l'estradiol tritié (également à 5mM final) avec un niveau de sensibilité plus important, grâce à l'activité spécifique plus élevée, et un niveau de liaison non spécifique beaucoup plus bas (23 % de la liaison non spécifique contre 62 % pour le ligand tritié). La liaison non spécifique aux protéines de transport est diminuée grâce à la présence des groupements en 11 beta et 17 alpha.

Ces caractéristiques apportent une amélioration au dosage du récepteur estrogène dans les tissus tumoraux provenant de biopsies en clinique.

L'analogue froid (16') est nécessaire pour l'estimation du niveau de non spécifique (excès d'hormone froide) dans ce type de dosage.

En outre, 16' constitue potentiellement un estrogène agoniste puissant.

### Exemple 5 : SYNTHESE DE DERIVES HALOGENES PAR L'INTERMEDIAIRE DES DERIVES TRIBUTYLETAIN

L'intérêt des dérivés tributylétain (15') et (13') et les analogues de la famille progestagènes est que ceux-ci sont des intermédiaires-clé dans la synthèse de stéroïdes dont un des hydrogènes terminaux de la fonction vinylique en 17 alpha est substitué par un halogène (F, Cl, Br, I, At) ou un autre atome dont la forme cationique peut substituer la fonction tributylétain.

On peut ainsi notamment préparer de nouveaux dérivés selon l'invention intéressants pour l'imagerie (¹⁸F) ou la thérapie (^{80m}Br ou ²¹¹At).

La synthèse de dérivés fluorés (froid ou ¹⁸F) s'effectue de la manière suivante : le dérivé tributylétain (10 µmole) dans CFCl₃ à - 78°C est mis en réaction avec 0,1 % F₂ (¹⁹F ou ¹⁸F) dans N₂ (50 µmole) pendant 40 minutes. Après passage sur HPLC, on obtient le produit pur.

Pour les dérivés ^{80m}Br ou ²¹¹At, on obtient les dérivés marqués respectivement à partir de Na ^{80m}Br ou Na ²¹¹At mis en réaction pendant 10 minutes avec le précurseur tributylétain dans l'éthanol et un volume égal d'une solution 2 : 1 (v/v) de 30 % H₂O₂ et acide acétique glacial. Après addition d'une solution de bisulfite, les dérivés halogénés sont extraits sur cartouche SPe C₁₈ (1ML, BAKER) et ensuite purifié par HPLC (µ BONDAPACK C₁₈ avec 50 à 60 % de EtOH dans H₂O comme phase mobile.

### Exemple 6 : LIGAND SPECIFIQUE DE RECEPTEUR PROGESTAGENE

La voie de synthèse des dérivés (E) et (Z) 11 beta-chlorométhyl 17 alpha-iodovinyl 17 beta-hydroxy 19-nor 4-androstène 3-one (respectivement (29') et (27') est décrite au schéma 2 ci-après. Il s'agit d'une modification du schéma 1 décrit précédemment pour les ligands spécifiques de récepteurs estrogènes. Le point de départ est le 3,11 beta-dihydroxy estra 1, 3, 5 (10) triène 17-one 17-éthylène Kétal (3').

L'étape 1' consiste en la méthylation en position C₃ de (3') par l'action de K₂CO₃ et CH₃I et chauffage à reflux pendant 24 heures dans l'acétone.

L'étape 2' consiste en l'oxydation en position C₁₁ par le pyridinium chlorochromate (PCC) selon un procédé analogue à celui décrit dans l'étape 5 du schéma 1.

L'étape 3' consiste en la préparation du dérivé silylé en position C₁₁ par le magnésien de chlorure de méthyltriméthylsilane selon la méthode de l'étape 6 du schéma 1, suivie par l'hydrolyse acide décrite dans l'étape 7 du schéma 1, elle-même suivie par la protection en C₁₇ par l'action de l'éthylène glycol en milieu acide comme décrit de façon analogue dans l'étape 8 du schéma 1. On obtient ainsi le composé (20').

L'étape 4' consiste en la préparation d'un dérivé borane fixé sur le carbone porté par C₁₁ et son oxydation par H₂O₂ en milieu basique pour donner le dérivé hydroxyméthyl en C₁₈ (21') selon la méthode analogue de l'étape 9 du schéma 1.

L'étape 5' consiste en la protection de l'hydroxyl attaché en C₁₁ par une fonction tétrahydropyranyle. La méthode de synthèse consiste en l'action de DHP (dihydropyrane) dans le THF en présence d'acide paratoluène sulfonique à température ambiante durant une nuit. Après évaporation des solvants, le dérivé (22') est extrait par CH₂Cl₂ après ajout d'H₂O.

L'étape 6' consiste en la réduction par réaction de Birch du composé (22') suivie par acidification par HCl pour libérer la cétone alpha, beta conjuguée du dérivé (23'), simultanément la fonction en C₁₁ est déprotégée. Le mode opératoire est le suivant : 0,75 g de Lithium sont dissous dans 75 ml de NH₃ liquide, on obtient une coloration bleue foncée. Pendant la réduction, la température est maintenue en dessous de - 50°C par un bain de carbobglace. On ajoute 3,1 g du stéroïde (22'). On agite le mélange pendant 1 h 30. Ensuite, on ajoute goutte à goutte 50 ml d'isopropanol et 20 ml d'éthanol. La décoloration est complète. On remonte lentement la température à 22°C et on laisse évaporer le NH₃ pendant une nuit.

Après extraction au CH₂Cl₂, on obtient environ 3 g d'une masse blanchâtre. On chauffe celle-ci à reflux 20 minutes dans un mélange de 100 ml de CH₃OH et 30 ml d'HCl 10 % à dans H₂O. Après extraction au CH₂Cl₂, on obtient 1,7 g d'un solide jaunâtre. Après chromatographie sur gel de silice avec 15 % d'acétone dans CH₂Cl₂, on obtient un produit pur en TLC et RMN.
TLC : Silice, CH₂Cl₂ (85 %) et acétone (15 %)
Rf = 0,20
Silice, CH₂Cl₂ (95 %) et méthanol (5 %)
Rf = 0,27

### Données spectroscopiques :

RMN (CDCl₃, TMS) ; déplacement chimique (ppm) 0.95 (s, 13CH₃), 3.7 (m, CHHOH), 3.95 (m, CHHOH), 5.85 (s, CH = C)
L'étape 7' consiste en la chlorination de la fonction carbonée en C₁₁ par N-chlorosuccinimide selon la méthode de l'étape 10 du schéma 1. On obtient le composé 24'.
Chlorination du 11 hydroxyméthyl Δ⁴ estrène 3,17 dione :
1. Sous N₂ sec, on ajoute une solution de 5,34 gr (20 mM) de triphénylphosphine dans 80 ml de THF sec à une solution de 2,71 gr (20 mM) de N chlorosuccinimide dans 80 ml de THF sec.
   La suspension obtenue est alors ajoutée sous forte agitation à une solution de 3,22 gr (10 mM) de 11 hydroxyméthyl Δ⁴ estrène 3,17 dione dans le 70 ml de THF sec.
   On laisse réagir sous N₂ et à température ambiante pendant la nuit.
2. Après l'évaporation du solvant, la dilution dans l'eau pure, l'extraction par le dichlorométhane, on isole 9,4 gr d'un solide brunâtre.
   Après une purification sur colonne de silice, on obtient 2,5 gr (8 mM), rdt : 80 % de cristaux jaunâtre. CCM SiO₂ CH₂Cl₂24/acétone 1 et cyclohexane 1/acétate d'éthyle 1.

L'étape 8' consiste en l'éthynylation de la fonction en C₁₇ alpha du composé 24' par la méthode au complexe éthylène diaminieacétylure de lithium selon la méthode analogue à l'étape 11 du schéma 1. On obtient le composé 25'.
1. Protection du C=O en position 3 (3 énamine).
   Sous N₂, 2,4 gr du composé obtenu à l'étape 7' sont dissous à chaud dans 24 ml de méthanol, puis on ajoute 1 ml de pyrrolidine. On maintient sous léger reflux pendant 30 min puis on laisse revenir à température ambiante pendant la nuit.
   Les cristaux sont filtrés et lavés avec du méthanol. On obtient 2,3 gr (rendement : 80 %) d'un produit gris jaunâtre. CCM SiO₂ CH₂Cl₂9/MeOhl.
2. Ethynylation du C=O en 17 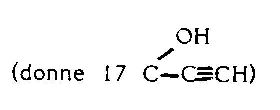 Sous N₂, 2,2 gr du composé obtenu à l'étape 8'1. (56 mM) sont dissous dans 40 ml de DMSO. On ajoute une solution de 3 gr (30 mM) du complexe lithium acétylure-éthylène-diamine dans 20 ml de DMSO. La réaction est stoppée après 2 h 30 en la versant dans un mélange eau/glace. L'extraction par l'acétate d'éthyle donne une huile orange qui est utilisée telle quelle dans l'étape suivante.
3. Hydrolyse de l'énamine en 3.
   L'huile brute obtenue ci-dessus est redissoute dans 50 ml de méthanol. A cette solution on ajoute 2,5 ml d'acide acétique, 2,5 gr d'acétate de sodium et 6 ml d'eau.
   Le mélange est reflué pendant 4 heures. On évapore alors le maximum de solvant puis on extrait par système eau-CH₂Cl₂. On obtient finalement 2,2 gr d'une huile rougeâtre.
4. Le produit brut est purifié sur une colonne de silice. Eluant : 2 % d'acétone dans le dichlorométhane.
   On obtient 0,6 gr de cristaux légèrement jaunes. Rendement total : 30 %. CCM SiO₂ CH₂Cl₂ 24 acétone 1 rf = 0,5.

L'étape 9' consiste en la préparation du dérivé (Z) tributylétain (26') par la méthode analogue à celle décrite dans l'étape 15 du schéma 1.

L'étape 10' consiste en la préparation du dérivé (Z) 11 beta-chlorométhyl 17 beta-hydroxy 17 alpha-(2-iodovinyl) 4-estrène 3-one (27').

Quand il s'agit d'un iode froid (¹²⁷I), on utilise la méthode de l'étape 16 du schéma 1, et quand il s'agit d'un marquage à l'¹²³I ou ¹²⁵I, on utilise la méthode de marquage également décrite pour le schéma 1.

L'étape 11' consiste en la préparation du dérivé (E) tributylétain (28') par la méthode décrite dans l'étape 13 du schéma 1.

L'étape 12' consiste en la préparation du dérivé (E) 11 beta-chlorométhyl 17 beta-hydroxy 17 alpha-(2-iodovinyl)-4-estrène 3-one (29').

Quand il s'agit d'un iode froid (¹²⁷I), on utilise la méthode de l'étape 14 du schéma 1, et quand il s'agit d'un marquage à l'¹²³I ou ¹²⁵I, on utilise la méthode de marquage également décrite à l'occasion du schéma 1.

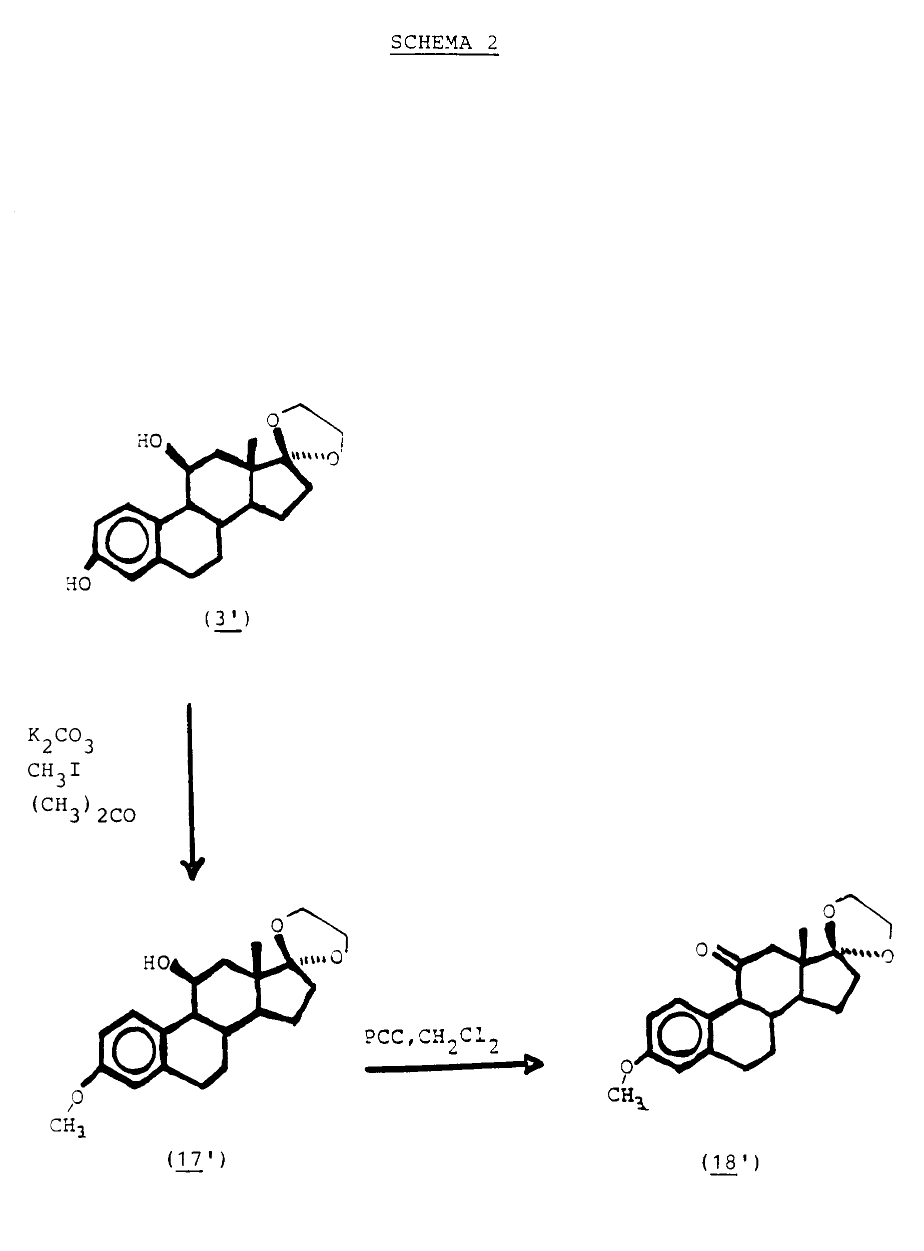

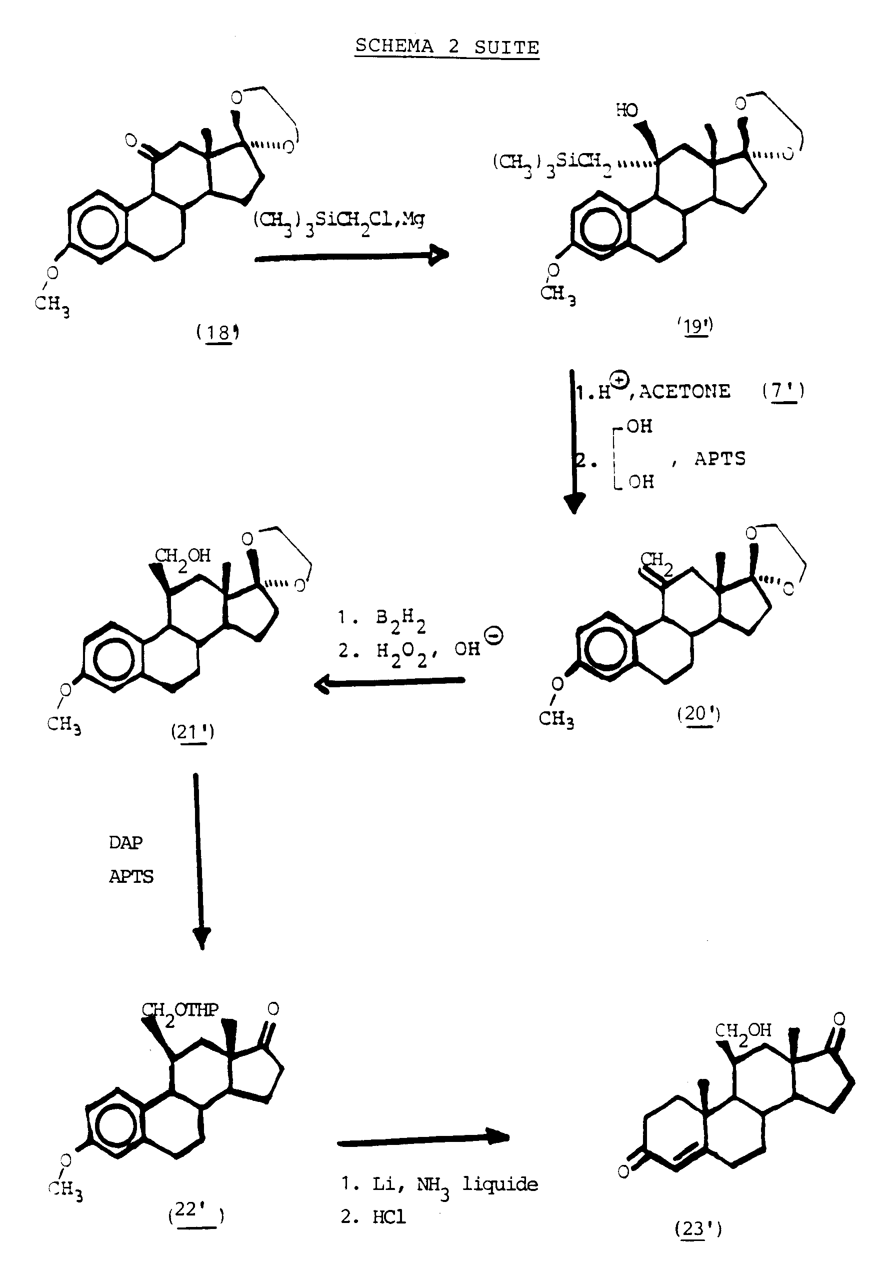

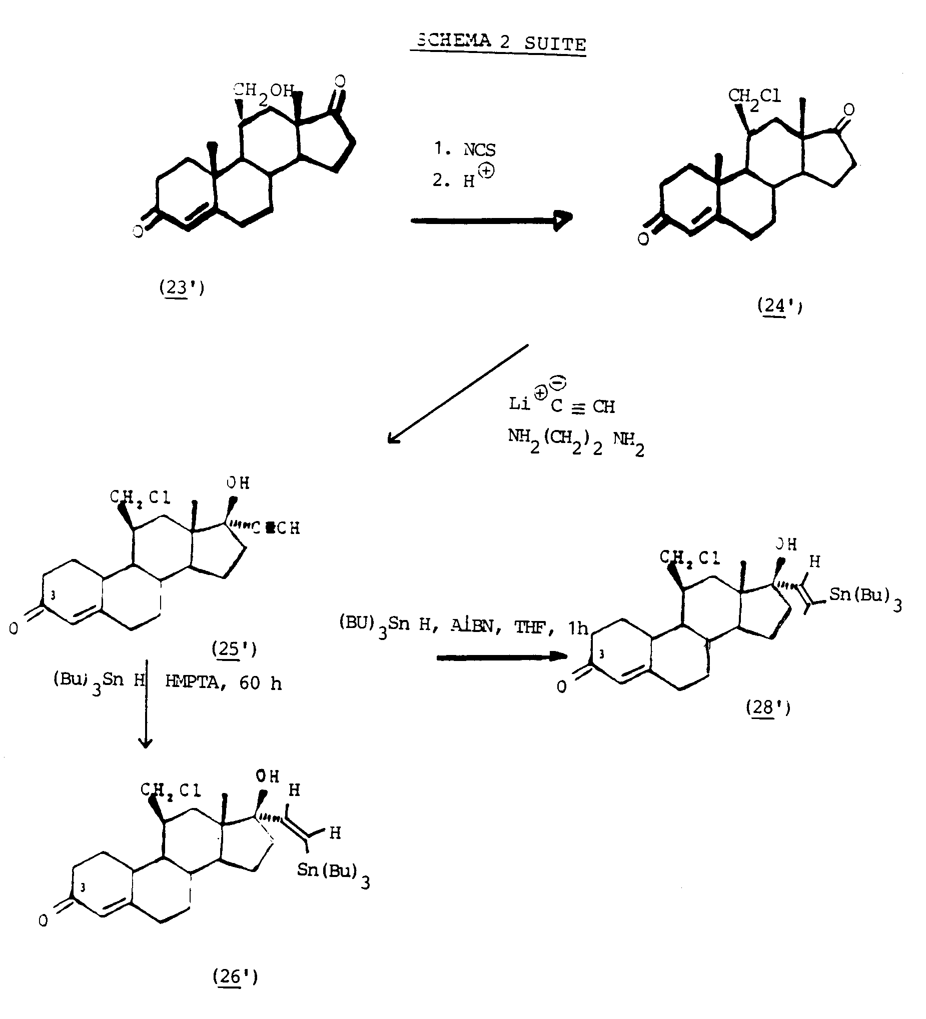

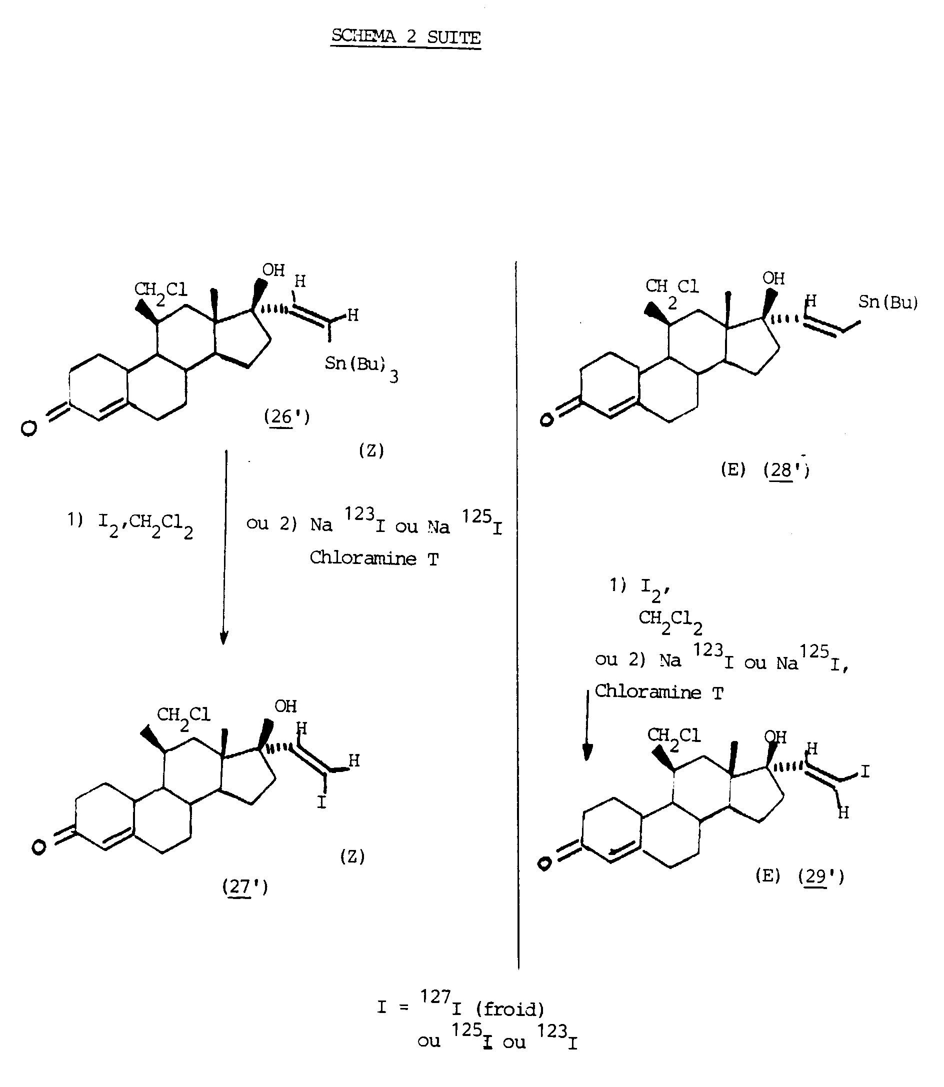

## Revendications (Revendications pour l'(les) Etat(s) contractant(s) suivant(s): AT, BE, CH, DE, FR, GB, IT, LI, LU, NL, SE)

1. Ligands spécifiques de récepteurs d'hormones stéroïdes estrogènes ou progestagènes ayant pour formule 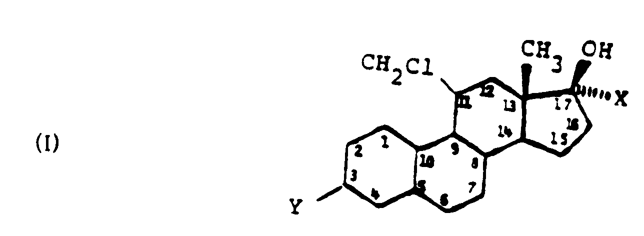 dans laquelle
X représente un groupement vinyl substitué sur la double liaison selon une isomérie Z par un halogène radioactif ou non radioactif, et
Y représente soit un groupe hydroxyl auquel cas le cycle sur lequel il est rattaché est un cycle aromatique, soit une fonction cétone auquel cas elle est conjuguée à une double liaison en C₄ - C₅.

2. Ligands spécifiques de récepteurs d'hormones stéroïdes selon la revendication 1 utiles pour la thérapie ciblée ou le dosage desdits récepteurs, caractérisés en ce qu'ils comportent un halogène non radioactif choisi parmi l'iode, le fluore, le brome et le chlore, substitué sur la double liaison du groupement vinyl rattaché en alpha de la position C₁₇ selon l'isomérie Z de la double liaison.

3. Ligands spécifiques de récepteurs d'hormones stéroïdes selon la revendication 1 utiles notamment pour la thérapie ciblée ou l'imagerie notamment du cancer ou le dosage desdits récepteurs, caractérisé en ce qu'ils comportent un halogène radioactif choisi parmi les isotopes radioactifs du fluore, de l'iode, du brome et de l'Astate, substitué sur la double liaison du groupement vinyl rattaché en alpha sur la position C₁₇ selon l'isomérie Z de la double liaison.

4. Ligands spécifiques de récepteurs d'hormones stéroïdes selon la revendication 3 utiles notamment pour la radiothérapie ciblée notamment du cancer, caractérisés en ce qu'ils comportent un isotope radioactif choisi parmi ¹²³I, ²¹¹At et ^{80m}Br substitué sur la double liaison du groupement vinyl, en alpha sur la position C₁₇ selon l'isomérie Z de la double liaison.

5. Ligands spécifiques de récepteurs d'hormones stéroïdes selon la revendication 3, utiles notamment pour l'imagerie médicale notamment du cancer, caractérisés en ce qu'ils comportent un isotope radioactif choisi parmi ¹²³I et ¹⁸F.

6. Ligands spécifiques selon la revendication 3, utiles notamment pour le dosage de récepteurs d'hormones stéroïdes, caractérisés en ce qu'ils comportent l'isotope ¹²⁵I substitué selon l'isomérie Z sur la double liaison du groupement vinyl en alpha de la position C₁₇.

7. Ligands selon l'une des revendications 1 à 6, caractérisés en ce que la fonction en position C₃ est un groupe hydroxyle attaché à un cycle aromatique.

8. Ligands selon l'une des revendications 1 à 6, caractérisés en ce que la fonction en position C₃ est une fonction cétone conjuguée à une double liaison en C₄-C₅.

9. Ligands selon la revendication 7, caractérisés en ce que c'est un 11 beta-chlorométhyl 17 alpha-halogénovinyl-estradiol.

10. Ligands selon la revendication 8, caractérisés en ce que c'est un 11 beta-chlorométhyl 17 alpha-halogénovinyl 17 beta-hydroxy-19-nor-4-androstène-3-one.

11. Ligand selon la revendication 7, caractérisé en ce que c'est un 11 beta-chlorométhyl 17 alpha-iodovinyl-estradiol d'isomérie Z.

12. Ligand selon la revendication 8, caractérisé en ce que c'est un 11 beta-chlorométhyl 17 alpha-iodovinyl 17 betahydroxy-19-nor-4-andorstèn-3-one d'isomérie Z.

13. A titre de produit nouveau utile notamment dans la synthèse des ligands spécifiques selon l'une des revendications précédentes un produit de formule 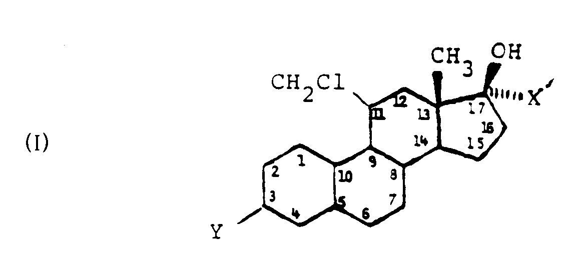 dans laquelle
X' représente un groupement vinyl en alpha de la position C₁₇ sur la double liaison duquel le groupe tributylstannyl est substitué selon une isomérie Z, et
Y a la signification donnée précédemment.

14. Procédé de synthèse des ligands selon l'une des revendications 1 à 13, caractérisé en ce que le dérivé halogéné est préparé à partir du produit selon la revendication 13.

## Revendications (Revendications pour l'(les) Etat(s) contractant(s) suivant(s): ES)

1. Procédé de préparation de ligands spécifiques de récepteurs d'hormones stéroïdes estrogènes ou progestagènes ayant pour formule 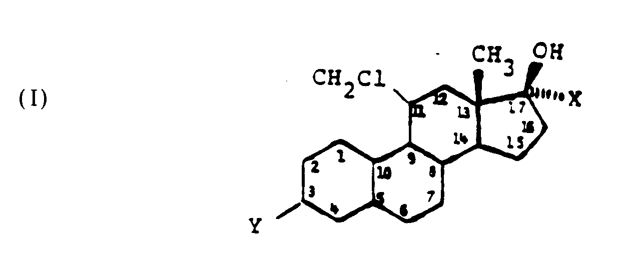 dans laquelle
X représente un groupement vinyl substitué sur la double liaison selon une isomérie Z par un halogène radioactif ou non radioactif, et
Y représente soit un groupe hydroxyl auquel cas le cycle sur lequel il est rattaché est un cycle aromatique, soit une fonction cétone auquel cas elle est conjuguée à une double liaison en C₄ - C₅,
à partir d'un produit de formule 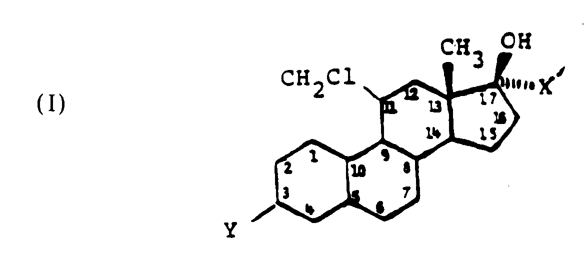 dans laquelle
X' représente un groupement vinyl en alpha de la position C₁₇ sur la double liaison duquel le groupe tributylstannyl est substitué selon une isomérie Z, et
Y a la signification donnée précédemment.

2. Procédé de préparation de ligands spécifiques de récepteurs d'hormones stéroïdes selon la revendication 1 caractérisé en ce qu'ils comportent un halogène non radioactif choisi parmi l'iode, le fluore, le brome et le chlore, substitué sur la double liaison du groupement vinyl rattaché en alpha de la position C₁₇ selon l'isomérie Z de la double liaison.

3. Procédé de préparation de ligands spécifiques de récepteurs d'hormones selon la revendication 1, caractérisé en ce qu'ils comportent un halogène radioactif choisi parmi les isotopes radioactifs du fluore, de l'iode, du brome et de l'Astate, substitué sur la double liaison du groupement vinyl rattaché en alpha sur la position C₁₇ selon l'isomérie Z de la double liaison.

4. Procédé de préparation de ligands spécifiques de récepteurs d'hormones stéroïdes selon la revendication 3 caractérisé en ce qu'ils comportent un isotope radioactif choisi parmi ¹²³I, ²¹¹At et ^{80m}Br substitué sur la double liaison du groupement vinyl, en alpha sur la position C₁₇ selon l'isomérie Z de la double liaison.

5. Procédé de préparation de ligands spécifiques de récepteurs d'hormones stéroïdes selon la revendication 3, caractérisé en ce qu'ils comportent un isotope radioactif choisi parmi ¹²³I et ¹⁸F.

6. Procédé de préparation de ligands spécifiques selon la revendication 3, caractérisé en ce qu'ils comportent l'isotope ¹²⁵I substitué selon l'isomérie Z sur la double liaison du groupement vinyl en alpha de la position C₁₇.

7. Procédé de préparation de ligands selon l'une des revendications 1 à 6, caractérisé en ce que la fonction en position C₃ est un groupe hydroxyle attaché à un cycle aromatique.

8. Procédé de préparation de ligands selon l'une des revendications 1 à 6, caractérisé en ce que la fonction en position C₃ est une fonction cétone conjuguée à une double liaison en C₄ - C₅.

9. Procédé de préparation de ligands selon la revendication 7, caractérisé en ce que c'est un 11 β-chlorométhyl 17 α-halogénovinylestradiol.

10. Procédé de préparation de ligands selon la revendication 8, caractérisé en ce que c'est un 11 β-chlorométhyl 17 α-halogénovinyl 17 β-hydroxy-19-nor-4-androstène-3-one.

11. Procédé de préparation de ligands selon la revendication 7, caractérisé en ce que c'est un 11 β-chlorométhyl 17 α-iodovinyl-estradiol d'isomérie Z.

12. Procédé de préparation de ligands selon la revendication 8, caractérisé en ce que c'est un 11 β-chlorométhyl 17 α-iodovinyl 17 β-hydroxy-19-nor-4-andorstèn-3-one d'isomérie Z.

13. Procédé de synthèse des ligands selon l'une des revendications 1 à 12, caractérisé en ce que le dérivé halogéné est préparé à partir du procédé selon la revendication 12.

## Claims (Claims for the following Contracting State(s): AT, BE, CH, DE, FR, GB, GR, IT, LI, LU, NL, SE)

1. Specific ligands for estrogen or progestagen steroid hormone receptors which have the formula 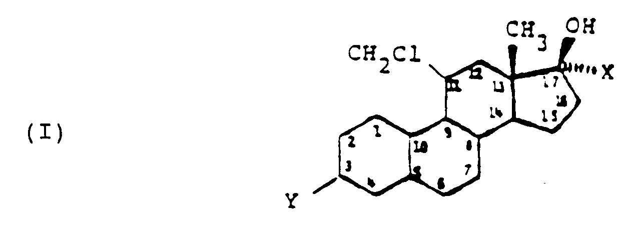 in which
X denotes a vinyl group substituted by a radioactive or nonradioactive halogen on the double bond according to a Z isomerism, and
Y denotes either a hydroxyl group, in which case the ring to which it is attached is an aromatic ring, or a ketone functional group, in which case it is conjugated with a double bond at C₄-C₅.

2. Specific ligands for steroid hormone receptors according to Claim 1, which can be used for the targeted therapy or the quantitative determination of said receptors, characterized in that they contain a nonradioactive halogen chosen from iodine, fluorine, bromine and chlorine, substituted on the double bond of the alpha-oriented vinyl group attached to the C₁₇ position according to the Z isomerism of the double bond.

3. Specific ligands for steroid hormone receptors according to Claim 1, which can be used especially for the targeted therapy or imagery, especially of cancer, or the quantitative determination of said receptors, characterized in that they contain a radioactive halogen chosen from the radioactive isotopes of fluorine, iodine, bromine and astatine, substituted on the double bond of the alpha-oriented vinyl group attached to the C₁₇ position according to the Z isomerism of the double bond.

4. Specific ligands for steroid hormone receptors according to Claim 3, which can be used especially for the targeted radiotherapy, especially of cancer, characterized in that they contain a radioactive isotope chosen from ¹²³I, ²¹¹At and ^{80m}Br substituted on the double bond of the vinyl group, with alpha orientation in position C₁₇ according to the Z isomerism of the double bond.

5. Specific ligands for steroid hormone receptors according to Claim 3, which can be used especially for the medical imagery, especially of cancer, characterized in that they contain a radioactive isotope chosen from ¹²³I and ¹⁸F.

6. Specific ligands according to Claim 3, which can be used especially for the quantitative determination of steroid hormone receptors, characterized in that they contain the isotope ¹²⁵I substituted according to the Z isomerism on the double bond of the alpha-oriented vinyl group in the C₁₇ position.

7. Ligands according to one of Claims 1 to 6, characterized in that the functional group in position C₃ is a hydroxyl group attached to an aromatic ring.

8. Ligands according to one of Claims 1 to 6, characterized in that the functional group in position C₃ is a ketone functional group conjugated with a double bond at C₄-C₅.

9. Ligands according to Claim 7, characterized in that they are an 11beta-chloromethyl-17alpha-halovinylestradiol.

10. Ligands according to Claim 8, characterized in that they are an 11beta-chloromethyl-17alpha-halovinyl-17beta-hydroxy-19-nor-4-androsten-3-one.

11. Ligand according to Claim 7, characterized in that it is an 11beta-chloromethyl-17alpha-iodovinylestradiol of Z isomerism.

12. Ligand according to Claim 8, characterized in that it is an 11beta-chloromethyl-17alpha-iodovinyl-17beta-hydroxy-19-nor-4-androsten-3-one of Z isomerism.

13. As a new product which can be used especially in the synthesis of specific ligands according to one of the preceding claims, a product of formula 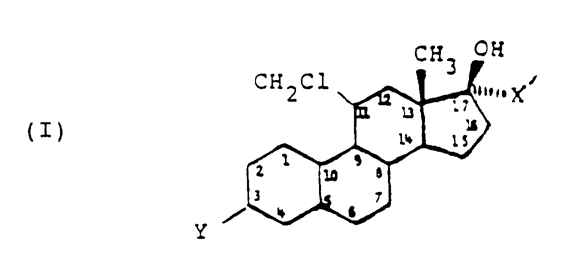 in which
X' denotes an alpha-oriented vinyl group in the C₁₇ position on the double bond of which the tributylstannyl group is substituted according to a Z isomerism, and
Y has the meaning given above.

14. Process for the synthesis of the ligands according to one of Claims 1 to 13, characterized in that the halogenated derivative is prepared from the product according to Claim 13.

## Claims (Claims for the following Contracting State(s): ES)

1. Process for the preparation of specific ligands for estrogen or progestagen steroid hormone receptors which have the formula 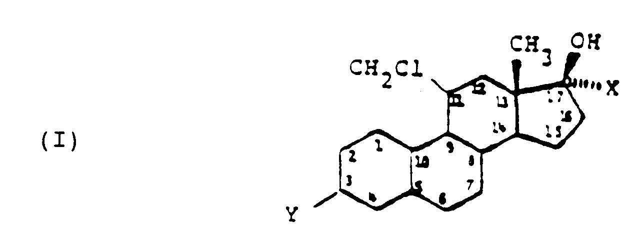 in which
X denotes a vinyl group substituted by a radioactive or nonradioactive halogen on the double bond according to a Z isomerism, and
Y denotes either a hydroxyl group, in which case the ring to which it is attached is an aromatic ring, or a ketone functional group, in which case it is conjugated with a double bond at C₄-C₅ from a product of formula 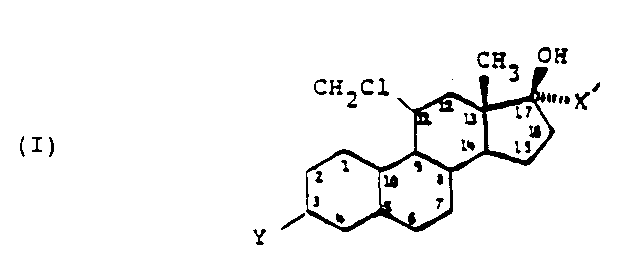
in which
X¹ denotes an alpha-oriented vinyl group in the C₁₇ position on the double bond of which the tributylstannyl group is substituted according to a Z isomerism, and
Y has the meaning given above.

2. Process for the preparation of specific ligands for steroid hormone receptors according to Claim 1, characterized in that they contain a nonradioactive halogen chosen from iodine, fluorine, bromine and chlorine, substituted on the double bond of the alpha-oriented vinyl group attached to the C₁₇ position according to the Z isomerism of the double bond.

3. Process for the preparation of specific ligands for hormone receptors according to Claim 1, characterized in that they contain a radioactive halogen chosen from the radioactive isotopes of fluorine, iodine, bromine and astatine, substituted on the double bond of the alpha-oriented vinyl group attached to the C₁₇ position according to the Z isomerism of the double bond.

4. Process for the preparation of specific ligands for steroid hormone receptors according to Claim 3, characterized in that they contain a radioactive isotope chosen from ¹²³I, ²¹¹At and ^{80m}Br substituted on the double bond of the vinyl group, with alpha orientation in position C₁₇ according to the Z isomerism of the double bond.

5. Process for the preparation of specific ligands for steroid hormone receptors according to Claim 3, characterized in that they contain a radioactive isotope chosen from ¹²³I and ¹⁸F.

6. Process for the preparation of specific ligands according to Claim 3, characterized in that they contain the isotope ¹²⁵I substituted according to the Z isomerism on the double bond of the alpha-oriented vinyl group in the C₁₇ position.

7. Process for the preparation of ligands according to one of Claims 1 to 6, characterized in that the functional group in position C₃ is a hydroxyl group attached to an aromatic ring.

8. Process for the preparation of ligands according to one of Claims 1 to 6, characterized in that the functional group in position C₃ is a ketone functional group conjugated with a double bond at C₄-C₅.

9. Process for the preparation of ligands according to Claim 7, characterized in that they are an 11β-chloromethyl-17α-halovinylestradiol.

10. Process for the preparation of ligands according to Claim 8, characterized in that they are an 11β-chloromethyl-17α-halovinyl-17β-hydroxy-19-nor-4-androsten-3-one.

11. Process for the preparation of a ligand according to Claim 7, characterized in that it is an 11β-chloromethyl-17α-iodovinylestradiol of Z isomerism.

12. Process for the preparation of a ligand according to Claim 8, characterized in that it is an 11β-chloromethyl-17α-iodovinyl-17β-hydroxy-19-nor-4-androsten-3-one of Z isomerism.

13. Process for the synthesis of the ligands according one of Claims 1 to 12, characterized in that the halogenated derivative is prepared from the product according to Claim 12.

## Patentansprüche (Patentansprüche für folgende(n) Vertragsstaat(en): AT, BE, CH, DE, FR, GB, GR, IT, LI, LU, NL, SE)

1. Liganden, die spezifisch sind für Östrogen- oder Progestagen-Steroidhormon-Rezeptoren und die Formel haben 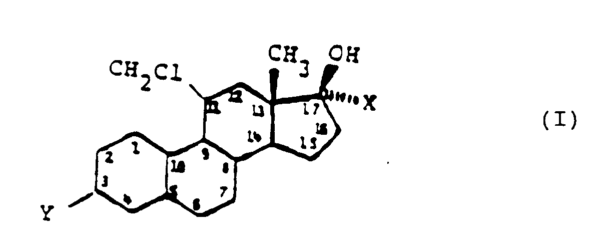 worin bedeuten:
X eine Vinylgruppe, die an der Doppelbindung entsprechend einer Z-Isomerie durch ein radioaktives oder nicht-radioaktives Halogen substituiert ist, und
Y entweder eine Hydroxylgruppe, wobei in diesem Falle der Ring, an den sie gebunden ist, ein aromatischer Ring ist, oder eine Ketonfunktion, wobei in diesem Falle diese zu einer Doppelbindung in C₄-C₅-Stellung konjugiert ist.

2. Für Steroidhormon-Rezeptoren spezifische Liganden nach Anspruch 1 für die Verwendung in der gezielten Therapie oder für die Bestimmung der genannten Rezeptoren, dadurch gekennzeichnet, daß sie ein nicht-radioaktives Halogen, ausgewählt aus der Gruppe Jod, Fluor, Brom und Chlor, enthalten, das an die Doppelbindung der Vinylgruppe gebunden ist, die gemäß der Z-Isomerie der Doppelbindung in α-Stellung der C₁₇-Position gebunden ist.

3. Für Steroidhormon-Rezeptoren spezifische Liganden nach Anspruch 1, die insbesondere für die gezielte Therapie oder die medizinische Bilderfassung insbesondere von Krebs oder für die Bestimmung der genannten Rezeptoren verwendbar sind, dadurch gekennzeichnet, daß sie ein radioaktives Halogen, ausgewählt aus der Gruppe der radioaktiven Isotope von Fluor, Jod, Brom und Astatin, enthalten, das an die Doppelbindung der Vinylgruppe gebunden ist, die entsprechend der Z-Isomerie der Doppelbindung in α-Stellung der C₁₇-Position gebunden ist.

4. Für Steroidhormon-Rezeptoren spezifische Liganden nach Anspruch 3, die insbesondere verwendbar sind für die gezielte Radiotherapie insbesondere von Krebs, dadurch gekennzeichnet, daß sie ein radioaktives Isotop, ausgewählt aus der Gruppe ¹²³J, ²¹¹At und ^{80m}Br enthalten, das an die Doppelbindung der Vinylgruppe gebunden ist, die entsprechend der Z-Isomerie der Doppelbindung in α-Stellung der C₁₇-Position gebunden ist.

5. Für Steroidhormon-Rezeptoren spezifische Liganden nach Anspruch 3, die insbesondere für die medizinische Bilderfassung, insbesondere von Krebs verwendbar sind, dadurch gekennzeichnet, daß sie ein radioaktives Isotop, ausgewählt aus der Gruppe ¹²³J und ¹⁸F enthalten.

6. Spezifische Liganden nach Anspruch 3, die insbesondere für die Bestimmung der Steroidhormon-Rezeptoren verwendbar sind, dadurch gekennzeichnet, daß sie das Isotop ¹²⁵J enthalten, das entsprechend der Z-Isomerie an die Doppelbindung der Vinylgruppe in α-Stellung der C₁₇-Position gebunden ist.

7. Liganden nach einem der Ansprüche 1 bis 6, dadurch gekennzeichnet, daß die Funktion in C₃-Position eine an einen aromatischen Ring gebundene Hydroxylgruppe ist.

8. Liganden nach einem der Ansprüche 1 bis 6, dadurch gekennzeichnet, daß die Funktion in C₃-Position eine Ketonfunktion ist, die zu einer Doppelbindung in C₄-C₅-Position konjugiert ist.

9. Liganden nach Anspruch 7, dadurch gekennzeichnet, daß es sich dabei handelt um ein 11β-Chloromethyl-17α-halogenovinyl-östradiol.

10. Liganden nach Anspruch 8, dadurch gekennzeichnet, daß es sich dabei handelt um ein 11β-Chloromethyl-17α-halogenovinyl-17β-hydroxy-19-nor-4-androsten-3-on.

11. Ligand nach Anspruch 7, dadurch gekennzeichnet, daß es sich dabei handelt um ein 11β-Chloromethyl-17α-jodovinyl-östradiol der Z-Isomierie.

12. Ligand nach Anspruch 8, dadurch gekennzeichnet, daß es sich dabei handelt um ein 11β-Chloromethyl-17α-jodovinyl-17β-hydroxy-19-nor-4-androsten-3-on der Z-Isomerie.

13. Neues Produkt, das insbesondere verwendbar ist für die Synthese der spezifischen Liganden nach einem der vorhergehenden Ansprüche und die Formel hat 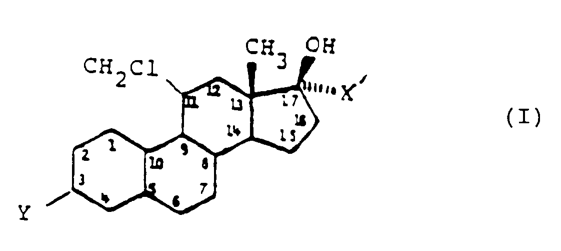 worin
X' steht für eine Vinylgruppe in α-Stellung der C₁₇-Position, an deren Doppelbindung die Tributylstannylgruppe gemäß einer Z-Isomerie gebunden ist, und
Y die oben angegebene Bedeutung hat.

14. Verfahren zur Synthese der Liganden nach einem der Ansprüche 1 bis 13, dadurch gekennzeichnet, daß das Halogenderivat hergestellt wird aus dem Produkt nach Anspruch 13.

## Patentansprüche (Patentansprüche für folgende(n) Vertragsstaat(en): ES)

1. Verfahren zur Herstellung von Liganden, die für Östrogen- oder Progestagen-Steroidhormon-Rezeptoren spezifisch sind und die Formel haben 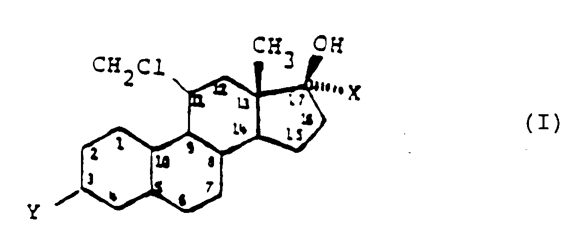 worin bedeuten:
X eine Vinylgruppe, die gemäß einer Z-Isomerie an der Doppelbindung durch ein radioaktives oder nicht-radioaktives Halogen substituiert ist, und
Y entweder eine Hydroxylgruppe, wobei in diesem Falle der Ring, an den sie gebunden ist, ein aromatischer Ring ist, oder eine Ketonfunktion, wobei in diesem Falle diese zu einer Doppelbindung in C₄-C₅-Position konjugiert ist,
aus einem Produkt der Formel 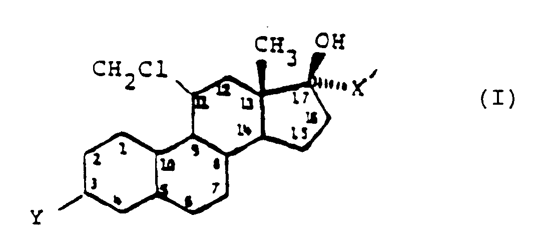 worin
X' steht für eine Vinylgruppe in α-Stellung der C₁₇-Position, an deren Doppelbindung gemäß einer Z-Isomerie die Tributylstannylgruppe gebunden ist, und
Y die oben angegebene Bedeutung hat.

2. Verfahren zur Herstellung von für Steroidhormon-Rezeptoren spezifische Liganden nach Anspruch 1, dadurch gekennzeichnet, daß sie ein nicht-radioaktives Halogen, ausgewählt aus der Gruppe Jod, Fluor, Brom und Chlor, enthalten, das an die Doppelbindung der Vinylgruppe gebunden ist, die gemäß der Z-Isomerie der Doppelbindung in α-Stellung der C₁₇-Position gebunden ist.

3. Verfahren zur Herstellung von für Hormon-Rezeptoren spezifische Liganden nach Anspruch 1, dadurch gekennzeichnet, daß sie ein radioaktives Halogen, ausgewählt aus der Gruppe der radioaktiven Isotope von Fluor, Jod, Brom und Astatin, enthalten, das an die Doppelbindung der Vinylgruppe gebunden ist, die entsprechend der Z-Isomerie der Doppelbindung in α-Stellung der C₁₇-Position gebunden ist.

4. Verfahren zur Herstellung von für Steroidhormon-Rezeptoren spezifische Liganden nach Anspruch 3, dadurch gekennzeichnet, daß sie ein radioaktives Isotop, ausgewählt aus der Gruppe ¹²³J, ²¹¹At und ^{80m}Br, enthalten, das an die Doppelbindung der Vinylgruppe in α-Stellung der C₁₇-Position gebunden ist gemäß der Z-Isomerie der Doppelbindung.

5. Verfahren zur Herstellung von für Steroidhormon-Rezeptoren spezifische Liganden nach Anspruch 3, dadurch gekennzeichnet, daß sie ein radioaktives Isotop, ausgewählt aus der Gruppe ¹²³J und ¹⁸F, enthalten.

6. Verfahren zur Herstellung von spezifischen Liganden nach Anspruch 3, dadurch gekennzeichnet, daß sie das Isotop ¹²⁵J enthalten, das gemäß der Z-Isomerie an die Doppelbindung der Vinylgruppe in α-Stellung zur C₁₇-Position gebunden ist.

7. Verfahren zur Herstellung von Liganden nach einem der Ansprüche 1 bis 6, dadurch gekennzeichnet, daß die Funktion in C₃-Stellung eine Hydroxylgruppe ist, die an einen aromatischen Ring gebunden ist.

8. Verfahren zur Herstellung von Liganden nach einem der Ansprüche 1 bis 6, dadurch gekennzeichnet, daß die Funktion in C₃-Stellung eine Ketonfunktion ist, die zu einer Doppelbindung in C₄-C₅-Position konjugiert ist.

9. Verfahren zur Herstellung von Liganden nach Anspruch 7, dadurch gekennzeichnet, daß es sich dabei handelt um ein 11β-Chloromethyl-17α-halogenovinyl-östradiol.

10. Verfahren zur Herstellung von Liganden nach Anspruch 8, dadurch gekennzeichnet, daß es sich dabei handelt um ein 11β-Chloromethyl-17α-halogenovinyl-17β-hydroxy-19-nor-4-androsten-3-on.

11. Verfahren zur Herstellung von Liganden nach Anspruch 7, dadurch gekennzeichnet, daß es sich dabei handelt um ein 11β-Chloromethyl-17α-jodovinyl-östradiol der Z-Isomerie.

12. Verfahren zur Herstellung von Liganden nach Anspruch 8, dadurch gekennzeichnet, daß es sich dabei han delt um ein 11β-Chloromethyl-17α-jodovinyl-17β-hydroxy-19-nor-4-androsten-3-on der Z-Isomerie.

13. Verfahren zur Synthese von Liganden nach einem der Ansprüche 1 bis 12, dadurch gekennzeichnet, daß das Halogenderivat hergestellt wird ausgehend von dem Verfahren nach Anspruch 12.
